(19) Europäisches Patentamt
European Patent Office
Office européen des brevets

(11) Veröffentlichungsnummer: **0 075 823**
**B1**

(12) # EUROPÄISCHE PATENTSCHRIFT

(45) Veröffentlichungstag der Patentschrift: **23.07.86**

(51) Int. Cl.⁴: **C 07 C 121/66**, A 61 K 31/275 // C07C121/66

(21) Anmeldenummer: **82108651.9**

(22) Anmeldetag: **18.09.82**

(54) **Verwendung von basisch substituierten Phenylacetonitrilen bei der vorbeugenden Bekämpfung von Krankheiten.**

(30) Priorität: **28.09.81 DE 3138488**

(43) Veröffentlichungstag der Anmeldung: **06.04.83 Patentblatt 83/14**

(45) Bekanntmachung des Hinweises auf die Patenterteilung: **23.07.86 Patentblatt 86/30**

(84) Benannte Vertragsstaaten: **AT CH DE FR GB IT LI NL SE**

(56) Entgegenhaltungen:
**DE-B-2 059 923**
**DE-B-2 112 648**

(73) Patentinhaber: **KNOLL AG, Knollstrasse, D-6700 Ludwigshafen (DE)**

(72) Erfinder: **Fleckenstein, Albrecht, Dr., Haus 6 F, D-7801 Bollschweil- St. Ulrich (DE)**

(74) Vertreter: **Karau, Wolfgang Dr., BASF Aktiengesellschaft Carl- Bosch- Strasse 38, D-6700 Ludwigshafen (DE)**

**EP 0 075 823 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft die neue Verwendung von basisch substituierten Phenylacetonitrilen bei der vorbeugenden Bekämpfung von Krankheiten.

Basisch substituierte Phenylacetonitrile sind bereits bekannt (vgl. DE—PS 1 154 810). Sie werden als Calciumantagonisten in der Therapie eingesetzt. Die Phenylacetonitrile sind auch in Form ihrer Antipoden wirksam (DE—PS 2 059 923 und 2 059 985). Weiter wird eine Kombination aus Verapamil und Chinidin zur Bekämpfung von Arrhythmien verwendet (DE—PS 2 112 648).

Es ist weiter bekannt, daß die häufigsten Komplikationen der Zuckerkrankheit im frühzeitigen Auftreten von Verkalkungsprozessen in der glatten Muskulatur der Arterienwände besteht. Dies führt zu Minderdurchblutung und einem oft hochgradigen Verschluß von Arterien in den Extremitäten, so daß Amputationen unvermeidbar werden. Darüberhinaus kommt es nicht selten auch zu schweren Zirkulationsstörungen in anderen Organen (Gehirn, Niere, Darm etc.). Besonders dramatische Veränderungen der Netzhaut-Gefäße können letztlich zu Erblindung führen. Eine andere, typische Komplikation des Diabetes, die ebenfalls auf einer Calcium-Überladung beruht, ist eine — oft frühzeitig auftretende — Linsen-Trübung (Katarakt). Eine am Pathomechanismus angreifende Therapie bestand für diese Indikationen bisher nicht: Abgesehen von der Kontrolle des Blutzuckers kamen bislang Vasodilatatoren — meist mit ungenügendem Erfolg — zur Anwendung. Katarakte wurden operativ beseitigt.

Es wurde nun gefunden, daß sich die genannten Komplikationen mit Hilfe einer bestimmten Gruppe von Calciumantagonisten weirtgehend verhindern lassen.

Gegenstand der Erfindung ist die Verwendung von basisch substituierten Phenylacetonitrilen der Formel

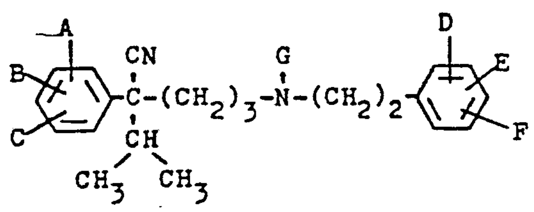

I

worin

A, B, C, D, E und F gleich oder verschieden sind und Wasserstoffatome oder $C_{1-4}$-Alkoxygruppen bedeuten und G ein Wasserstoffatom oder eine $C_{1-4}$-Alkylgruppe darstellt, sowie deren Salzen mit physiologisch verträglichen Säuren zur Herstellung eines Arzneimittels für die vorbeugende Bekämpfung von durch Calcinose von Gefäßwänden verursachten Arterienveränderungen.

Unter den bevorzugten Verbindungen der Formel I sind speziell Gallopamil und insbesondere Verapamil zu nennen. Gallopamil ist α-Isopropyl-α-[(N-methyl-N-homoveratryl)-γ-aminopropyl]-3,4,5-trimethoxyphenylacetonitril und Verapamil ist α-Isopropyl-α-[(N-methyl-N-homoveratryl)-γ-aminopropyl]-3,4-dimethoxyphenylacetonitril.

Als Salze kommen insbesondere diejenigen folgenden Säuren in Betracht: Schwefelsäure, Phosphorsäure, Essigsäure, Malonsäure, Bernsteinsäure, Fumarsäure, Maleinsäure, Zitronensäure, Weinsäure, Milchsäure, Diaminosulfonsäure und insbesondere Salzsäure.

Als durch Calcinose von Gefäßwänden verursachte Arterienveränderungen (Mönckeberg-Typ der Arteriosklerose) sind insbesondere zu nennen:

Arterienveränderungen bei Diabetikern, Gefäßschäden des Diabetikers am Auge, Linsentrübungen auf der Basis einer Calcium-Überladung, diabetische Katarakte.

Gemaß der Erfindung wird es zum ersten Mal möglich, die Krankheiten vorbeugend bei Warmblütern mit Erfolg medikamentös zu behandeln, wie folgende Versuche zeigen:

Wistar-Ratten im Alter von 2 Monaten erhielten zur Erzeugung eines Diabetes mellitus intravenös 80 mg/kg Alloxan. Nach Zerstörung der Insulin-produzierenden ß-Zellen in Pancreas stieg der Blutzuckerwert bei den Tieren von 100 mg% auf etwa 420 mg%. Dieser Wert blieb während einer Beobachtungszeit von 6 1/2 Monaten praktisch konstant. Während der Beobachtungszeit erhielt ein Teil der Tiere täglich 25 mg/kg Verapamilhydrochlorid mit einer Schlucksonde. Bei den nicht mit Verapamil behandelten Tieren kam es in dieser Zeit bei 63% der Tiere zu einem völligen Verlust der Transparenz der Augenlinse, während sich bei den behandelten Tieren nur in 4% der Fälle ein Katarakt bildete.

Ohne Verapamil stieg der Calcium-Gehalt der Linse diabetischer Ratten vom Normalwert nichtdiabetischer Kontrolltiere (= 1,08(±)0,02) auf 10,89 (± 0,85) mmol/kg Trockengewicht an. Dagegen verblieb mit Verapamil der mittlere Calcium-Gehalt der Linsen diabetischer Tiere in dem niedrigeren Bereich von 3,46 (± 0,61) mmol/kg Trockengewicht.

Parallel mit der Verhütung der diabetischen Linsen-Calcinose konnte bei den gleichen Tieren auch der abnorme Anstieg der Calcium-Werte in der Wand der Aorta und der Arteria mesenterica superior (Darmarterie) durch die Verapamil-Behandlung verhindert werden.

Mit der Verhinderung des Calcium-Anstiegs in den Arterien-Wänden wurden auch die elektronenoptisch — hauptsächlich in der Media — nachweisbaren degenerativen Prozesse verhütet.

Entsprechende Ergebnisse lassen sich beispielsweise mit Gallopamil erhalten.

Die Phenylacetonitrile der Formel I werden in einer Dosis von etwa 100 bis 1000 mg pro Patient und Tag verabfolgt. Die genaue Dosis muß für die einzelnen Substanzen jeweils exakt ermittelt werden. Sie beträgt beim Verapamilhydrochlorid 240 bis 480 mg und beim Gallopamilhydrochlorid 100 bis 150 mg pro Patient (80 kg) und Tag. Diese Daten gelten nur, wenn keine weiteren herzaktiven Substanzen verabfolgt werden. Die gleichzeitige Gabe von ß-Rezeptorenblockern ist zu vermeiden. Die Verbindungen der Formel I lassen sich gewünschtenfalls auch mit Substanzen zusammen applizieren, die den Blutzuckerspiegel herabsetzen.

Es ist äußerst überraschend, daß man mit den Verbindungen der Formel I durch Calcinose von Gefäßwänden verursachte Arterienveränderungen verhindern kann. Versuche, denselben Effekt mit anderen Calciumantagonisten, wie Nifedipin oder Niludipin zu erzielen, blieben erfolglos.

Die Verbindungen der Formel I werden für die neue Anwendung oral verabfolgt. Hierzu werden sie in die üblichen galenischen Applikationsformen — wie Tabletten oder Dragees — gebracht. Besonders geeignet sind Depotformen, die in bekannter Weise hergestellt werden können.

**Patentanspruch**

Verwendung von basisch substituierten Phenylacetonitrilen der Formel

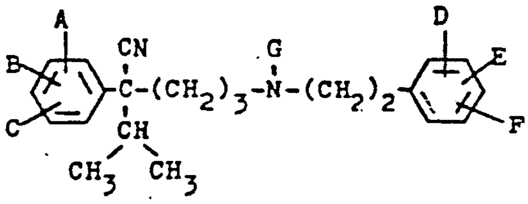

I

worin

A, B, C, D, E und F gleich oder verschieden sind und Wasserstoffatome oder $C_{1-4}$-Alkoxygruppen bedeuten und G ein Wasserstoffatom oder eine $C_{1-4}$-Alkylgruppe darstellt, sowie deren Salzen mit physiologisch verträglichen Säuren zur Herstellung eines Arzneimittels für die vorbeugende Bekämpfung von durch Calcinose von Gefäßwänden verursachten Arterienveränderungen.

**Revendication**

Utilisation de phénylacétonitriles à substitution basique de formule

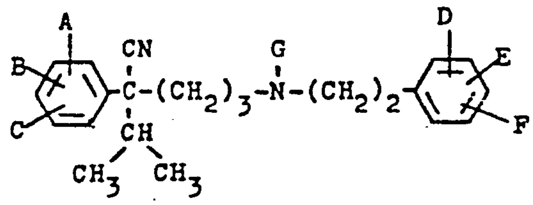

I

où

A, B, C, D, E, et F sont identiques ou différents et représentent des atomes d'hydrogène ou des groupes alcoxy en $C_{1-4}$ et G représente un atome d'hydrogène ou un groupe alkyle en $C_{1-4}$, ainsi que leurs sels d'acides acceptables physiologiquement pour la préparation d'un médicament pour la lutte prophylactique contre les altérations des artères provoquées per la calicinose de leurs parois.

**Claim**

The use of a phenylacetronitrile carrying basic substituents, of the formula

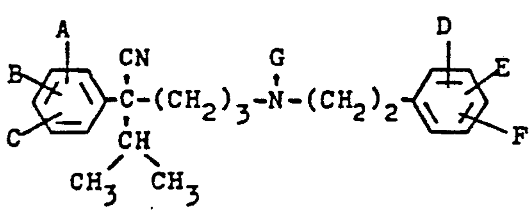

I

where A, B, C, D, E and F are identical or different and each is hydrogen or $C_1$—$C_4$-alkoxy, and G is hydrogen or $C_1$—$C_4$-alkyl, or of a salt thereof with a physiologically acceptable acid, for the preparation of a drug for the prophylaxis of arterial changes caused by calcinosis of the vascular walls.